# EUROPEAN PATENT SPECIFICATION

(11) **EP 1 292 290 B1**
(45) Date of publication and mention of the grant of the patent: **19.04.2006**
(21) Application number: 01956495.4
(22) Date of filing: 20.06.2001
(51) Int. Cl.: A61K 31/409, A61K 31/704, A61P 35/00

(54) **PHARMACEUTICAL COMPOSITIONS COMPRISING ACRYLOYL DISTAMYCIN DERIVATIVES AND TOPOISOMERASE I AND II INHIBITORS**
PHARMAZEUTISCHE ZUSAMMENSETZUNGEN DIE ACRYLOYL-SUBSTITUIERTE DISTAMYCIN-DERIVATE UND TOPOISOMERASE I UND II INHIBITOREN ENTHALTEN
THERAPIE COMBINEE CONTRE DES TUMEURS A BASE DE DERIVES DE DISTAMYCINE ACRYLOYLE ET D'INHIBITEURS DE LA TOPOISOMERASE I ET II

(30) Priority: 23.06.2000 GB 0015444
(43) Date of publication of application: 19.03.2003
(73) Proprietor: Nerviano Medical Sciences S.r.l., 20014 Nerviano MI (IT)
(72) Inventor: GERONI, Maria, Cristina, Rosa, I-20149 Milano (IT); COZZI, Paolo, I-20133 Milano (IT); BERIA, Italo, I-20014 Nerviano (IT)
(74) Representative: Modiano, Micaela Nadia
(86) International application number: PCT/EP2001/007059
(87) International publication number: WO 2001/097789

(56) References cited:
- WO-A-98/04524
- WO-A-98/21202
- WO-A-99/34796
- WO-A-99/50266
- SOLA F ET AL: "The antitumor efficacy of cytotoxic drugs is potentiated by treatment with PNU 145156E, a growth-factor-complexing molecule" CANCER CHEMOTHERAPY AND PHARMACOLOGY, SPRINGER VERLAG, BERLIN, DE, vol. 43, no. 3, 1999, pages 241-246, XP002104215 ISSN: 0344-5704
- ZOU J P ET AL: "Distamycin A derivatives potentiate tumor-necrosis factor activity via the modulation of tyrosine phosphorylation" INTERNATIONAL JOURNAL OF CANCER, NEW YORK, NY, US, vol. 72, no. 5, 1997, pages 810-814, XP002104217 ISSN: 0020-7136
- TAGLIABUE G ET AL: "Combination of the New Minor Groove Alkylator Tallimustine and Melphalan" EUROPEAN JOURNAL OF CANCER, PERGAMON PRESS, OXFORD, GB, vol. 33, no. 2, February 1997 (1997-02), pages 284-287, XP004282511 ISSN: 0959-8049
- COZZI P ET AL: "Cytotoxic alpha-bromoacrylic derivatives of distamycin analogues modified at the amidino moiety" BIOORGANIC & MEDICINAL CHEMISTRY LETTERS, OXFORD, GB, vol. 10, no. 11, June 2000 (2000-06), pages 1273-1276, XP004200573 ISSN: 0960-894X
- COZZI P ET AL: "Cytotoxic halogenoacrylic derivatives of distamycin A" BIOORGANIC & MEDICINAL CHEMISTRY LETTERS, OXFORD, GB, vol. 10, no. 11, June 2000 (2000-06), pages 1269-1272, XP004200572 ISSN: 0960-894X

## Description

The present invention relates to the field of cancer treatment and provides an antitumor composition comprising a substituted acryloyl distamycin derivative, more particularly an α-bramo- or α-chloro-acryloyl distamycin derivative, and a topoisomerase inhibitor of type I or II, having a sinergistic antineoplastic effect

Distamycin A and analogues thereof, hereinafter referred to as distamycin and distamycin-like derivatives, are known in the art as cytotoxic agents useful in antitumor therapy.
Distamycin A is an antibiotic substance with antiviral and antiprotozoal activity, having a polypyrrole framework [*Nature* 203:1064 (1964); *J Med. Chem.* 32:774-778 (1999)].
The international patent applications WO 90/11277, WO 98/04524, WO 98/21202, WO 99/50265, WO 99/50266 and WO 01/40181 (claiming priority from British patent application. No. 9928703.9), all in the name of the applicant itself and herewith incorporated by reference, disclose acryloyl distamycin derivatives wherein the amidino moiety of distamycin is optionally replaned by nitrogen-contairning ending groups such as, for instance, cyanamidino, N-methylamidino, guanidino, carbamoyl, amidoxime, cyano and the like, and/or wherein the polypyrrole framework of distamycin, or part of it, is replaced by varying carbocyclio or heterocyclic moieties.

In particular WO 98/04524 relates to some distamycin derivatives, to a process for preparing them, to pharmaceutical compositions containing them and to their use in therapy, particularly as antitumor and antiviral agents. The derivatives disclosed in WO 98/04524 belong to a new class of distamycin derivatives wherein the distamycin formyl group is substituted by an acryloyl moiety and the amidine group is substituted by different nitrogen-containing end-groups. WO 98/04524 shows that these derivatives display valuable biological properties.

WO 99/50266 relates to alkylating antitumor agents analogous to Distamycin A, to a process for their preparation, to pharmaceutical compositions containing them and to their use as therapeutic agents. The derivatives disclosed in WO 99/50266 belong to a new class of distamycin derivatives wherein the distamycin formyl group is substituted by benzoheterocyclic rings bearing alkylating groups and the amidino moiety is substituted by different nitrogen-containing ending-groups. WO 99/50266 shows that these derivatives display valuable biological properties.

WO 99/21202 relates to distamycin derivatives, to a process for preparing them, to pharmaceutical compositions containing them and to their use in therapy, particularly as antitumor and antiviral agents. The derivatives disclosed in WO 98/21202 belong to a new class of diatamycin derivatives, wherein the distamycin formyl group is substituted by a benzoheterocyclic ring bearing an alkylating group. WO 98/21202 shows that these derivatives display valuable biological properties.

The present invention provides, in a first aspect, a pharmaceutical composition for use in antineoplastic therapy in mammals, including humans, comprising a pharmaceutically acceptable carrier or excipient,
- an acryloyl distamycin derivative of formula (I): 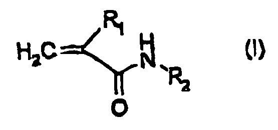 wherein:
   R₁ is a bromine or chlorine atom;
   R₂ is a distamycin or distamycin-like framework of formula (II) as defined in claim 1 or a pharmaceutically acceptable salt thereof; and
- an antineoplastic topoisomerase inhibitor of type I or II.

The present invention includes, within its scope, the pharmaceutical compositions comprising any of the possible isomers covered by the compounds of formula (I), both considered separately or in admixture, as well as the metabolites and the pharmaceutically acceptable bio-precursors (otherwise known as pro-drugs) of the compounds of formula (I).
In the present description, unless otherwise specified, with the term distamycin or distamycin-like framework R₂ we intend any moiety of formula (II) structurally closely related to distamycin itself, for instance by optionally replacing the ending amidino moiety of distamycin and/or its polypyrrole framework, or part of it.

Topoisomerase I and II inhibitors are known in the art as described in various scientific publications.
The main representatives for topoisomerase I inhibitors are camptothecin derivatives such as, for instance, CPT-11, Topotecan, 9-amino-camptothecin, 9-nitro-camptothecin and 10,11-methylenedioxy-camptothecin.
Among the topoisomerase II inhibitors are, in particular, the anthracycline derivatives such as doxorubicin, daunorubicin, epirubicin, nemorubicin and idarubicin: the podophyllotoxin compounds etoposide and teniposider, the anthraquinone derivatives like mitoxantrone and losoxantrone; the acridine derivatives like amsacrine and dactinomycin , Sen, for a reference, Cancer, Principles and Practice of Oncology, for a reference, Cancer, Principles and Practice of Ontology, Lippincott-Raven Ed. (1997), 452-467.

According to a preferred embodiment of the invention, herewith provided are the above pharmaceutical compositions wherein the topoisomerase inhibitors are topoisomerase II inhibitors, in particular doxorubicin and stoposide.

According to another preferred embodiment of the invention, herewith provided are the above pharmaceutical compositions wherein, within the acryloyl distamycin derivative of formula (I), R₁ has the above reported meanings and R₂ is a group of formula (II) below. 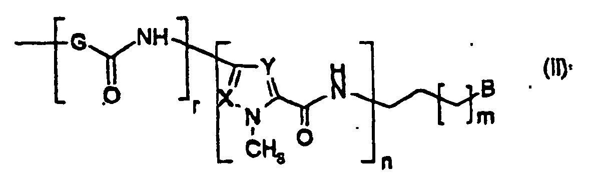 wherein
m is 0;
n is 4;
r is 0;
X and Y are both a CH group;
B is the group 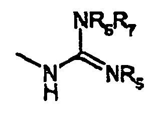 wherein R₅, R₆ and R₇, the same or different, are hydrogen or C₁-C₄ alkyl.
In the present description, unless otherwise specified, with the term C₁-C₄ alkyl or alkoxy group we intend a straight or branched group selected from methyl, ethyl, n-propyl, isopropyl, n-butyl isobutyl, sec-butyl, tert-butyl, methoxy, ethoxy, n-propoxy, isopropoxy, n-butoxy, isobutoxy, aeo-butoxy or tert-butoxy.

Even more preferred are the pharmaceutical compositions of the invention comprising the above acryloyl dietamycin derivative of formula (I) wherein R₁ is bromine or chlorine; R₂ is the above group of formula (II) wherein r is 0, m is 0 n is 4 and B has the above reported meanings.
Still more preferred, within this class, are the pharmaceutical compositions comprising the compounds of formula (I) wherein R₁ is bromine or chlorine, R₂ is the above group of formula (II) wherein r is 0, m is 0 n is 4, X and Y are both CG groups and B is 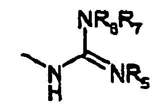 wherein R₅, R₆ and R₇, the same or different, are hydrogen or C₁-C₄ alkyl.

Pharmaceutically acceptable salts of the compounds of formula (I) are those with pharmaceutically acceptable inorganic or organic acids such as, for instance, hydrochloric, hydrobromic, sulfuric, nitric, acetic, propionic, succinic, malonic, citric, tartaric, methanesulfonic p-toluenesulfonic acid and the like.

Examples of prefened acryloyl distamycin derivatives of formula (I), within the compositions object of the invention, optionally in the form of pharmaceutically acceptable salts, preferably with hydrochloric acid, are:
1. N-(5-{[(5-{[(5-{[(2-{[amino{[imino)methyl]amino]ethyl)amino]carbonyl}-1-methyl-1H-pyrrol-3-yl)amino]carbonyl}-1-methyl-1H-pyrrol-3-yl)amino]carbonyl}-1-methyl-1H-pyrol-3-yl)-4-[(2-bromoacryloyl)amino]-1-methyl-1H pyrrol-2-carboxamide hydrochloride;
2. N-(5-{[(5-{[5-{[(2-{[amino(imino)methyl]amino )ethyl)amino]carbonyl}-1-methyl-1H-pyrrol-3-yl)amino]carbonyl}-1-methyl-1H-pyrrol-3-yl)amino]carbonyl}-1-methyl-1H-pyrrol-3-yl)-4-[(2-chloroacryloyl)amino)]-1-methyl-1H-pyrrolo-2-carboniamide hydrochloride;

The above compounds of formula (I), either specifically identified as such or by means of the general formula, are known or easily prepared according to known methods as reported, for instance, in the afore mentioned international patent applications WO 90/11277. WO 98/04524, WO 98/21202, WO 99/50265, WO 99/50266 and WO 01/40181.

The present invention further provides a product comprising an acryloyl distamycin derivative of formula (I), as defined above, and an antineoplastic topoisomerase I or II inhibitor, as a combined preparation for simultaneous, separate or sequential use in antitumor therapy.

A further aspect of the present invention is to provide the use of the above acryloyl distamycin derivative of formula (I) and an antineoplastic topoisomerase I or II inhibitor, in amounts effective to produce a synergistic antineoplastic effect in the preparation of a medicament for use in combination therapy for treating a mammal, including humans, suffering from a neoplastic disease state.

Even though it does not represent an aspect of the present invention, it is noted that a combined preparation comprising an antineoplastic topoisomerase I or II inhibitor and an acryloyl dystamycin derivative of formula (I), as defined above, in amounts effective to produce a synergistic antineoplastic effect is suitable for lowering the side effects caused by antineoplastic therapy with an antineoplastic agent in a mammal in need thereof, including humans.

By the term "synergistic antineoplastic effect", as used herein, it is meant the inhibition of the growth tumor, preferably tha complete regression of the tumor, by administering an effective amount of the combination, comprising an acryloyl distamycin derivative of formula (I) and a topoisomeraze I or II inhibiter to mammals, including humans By the term "administered" or "administering", as used herein, it is meant parenteral and/or oral administration; the term"parentaral" means intravenous, substanaous and intramuscular administration.

In the use of the present invention, the acryloyl distamycin derivative may be administered simultansously with the compound having topoisomerase I or II inhibitory activity, for example with a compound of the camptothecin, anthracycline, mitoxantrone, epipodophyllotoxin, or anridine class. Alternatively, both compounds may be administered sequentially in either order.
In this respect, it will be appreciated that the actual preferred use and order of administration will vary according to, inter alia, the particular formulation of the acryloyl distamycin of formula (I) being used, the particular formulation of the topoisomerase I or II inhibitor being used, for instance the camptothecins such as CPT-11, topotecan, 9-AC; the anthracyclines such as doxorubicin, damorubicin, epirubicin, idarubicin, nemorubicin; the anthraquinones such as mitoxantrone and Iosoxantrone; the epipodophyllotoxins such as etoposide, teniposide; the acridine derivatives such as amascrine and dactinomycin the particular tumor model being treated as well as the particular host being treated.
To administer the acryloyl distamycin derivative of formula (I), according to the use of the invention, the course of the therapy generally employed comprises closes varying from about 0.05 to about 100 mg/m² of body surface area and, more preferably, from about 0.1 to about 50 mg/m² of body surface area.
For the administration of the topoisomerase I or II inhibitor, according to the use of the invention, the course of therapy generally employed comprises
- when administering camptothecins: doses varying from about 1 to about 1000 mg/m² of body surface area and, more preferably, from about 10 to about 500 mg/m² of body surface area;
- when administering anthracyclines: doses varying from about 0.1 to about 1000 mg/m² of body surface area and, more preferably, from about 0.3 to about 500 mg/m²of body surface area;
- when administering epipodophyllotoxins: doses varying from about I to about 500 mg/m² of body surface area and, more preferably, from about 10 to about 400 mg/m² of body surface area;
- when administering anthraquinones: doses varying from about I to about 300 mg/m² of body surface area and, more preferably, from about 5 to about 100 mg/m² of body surface area.
- when administering acridine and dactynomicin derivatives doses varying from about 1 to about 1000 mg/m² of body surface area end, more preferably, from about 10 to about 500 mg/m² of body surface area.

The use of the present invention is particularly suitable for treating breast, ovary, lung, colon, kidney, stomach, pantreas, liver, melanoma, leukemia and brain tumors in mammals, including humans.

In a further aspect, the present invention is directed to the preparation of a pharmaceutical composition comprising an effective amount of an acryloyl distamycin derivative of formula (I), as defined above, and an antineoplastic topoisomerase I or II inhibitor, in the preparation of a medicament for use in the prevention or treatment of metastasis or in the treatment of tumors by inhibition of angiogenesis.

As stated above, the effect of an acryloyl distamycin derivative of formula (I) and a topoisemerase I or II inhibitor, such as an etoposide derivative, in significantly increased without a parallel increase of toxicity. In other words, the combined therapy of the present invention enhances the antitumoral effects of the acryloyl distamycin derivative and of the topoisomerase I or II inhibitor and, hence, provides the most effective and least toxic treatment for tumors.

The superadditive effects of the combined preparations of the invention are shown, for instance, by the following *in vivo* tests, which are intended to illustrate the present invention without posing any limitation to it.

Table 1 shows the antileukemic activity on disseminated L1210 murine leukemia obtained by combining N-(5-{[(5-{[(5-{[(2-{[amino(imino)methyl]amino} ethyl)amino]caibonyl)-1-methyl-1H-pyrrol-3-yl)amino]carbonyl}-1-methyl-1H-pyrrol-3-yl)amino]carbonyl}-1-methyl-1H-pyrrol-3-yl)-4-[(2-bromoacryloyl)amino]-1-methyl-1H-pyrrole-2-carboxamide hydrochloride, as a representative compound of formula (I) - internal code PNU 166196, with doxorubicin. At the dose of 10 mg/kg of doxorubicin alone (day +1 after tumor injection and 2 hours after PNU 166196 administration) and at the dose of 0.52 mg/kg of PNU-166196 alone (day+1) were associated, without toxicity, ILS% values of 58 and 33, respectively. Combining doxorubicin and PNU 166196 at the same doses with the same schedule, an increase of activity with ILS% value of 100 was observed, thus indicating a synergistic effect.

**Table 1: Antileukemic activity against disseminated L1210¹ murine leukemia of an acryloyl distamycin derivative (I) in combination with doxorubicin**

| Compound | Treatment² schedule | Dose (mg/kg/day) | ILS%³ | Tox⁴ |
|---|---|---|---|---|
| PNU 166196 | iv+1 | 0.52 | 33 | 0/10 |
| Doxorubicin | iv+1(*) | 10 | 58 | 0/10 |
| PNU 166196 + Doxorubicin | iv+1 | 0.52 + 10 | 100 | 0/10 |
| | iv+1(*) | | | |

| | | | | |
|---|---|---|---|---|
| 1. L1210 leukemia cells (10⁵/mouse CD2F1) are injected IV on Day 0. | | | | |
| 2. Treatment is given IV. | | | | |
| 3. Increase in life span: [(median survival time of treated mice/median survival time of controls) x 100] -100. | | | | |
| 4. Number of toxic deaths/number of mice. | | | | |
| (*) doxorubicin was administered 2 h after PNU 166196 administration. | | | | |

For these experiments, PNU 166196 and doxorubicin were solubilized in water for injection.

## Claims

1. A pharmaceutical composition comprising a pharmaceutically acceptable carrier or excipient and, as active ingredient, .
- an acryloyl distamycin derivative of formula (I): 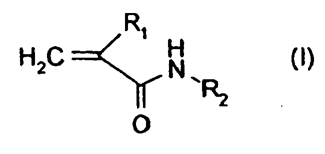 wherein:
R₁ is a bromine or chlorine atom;
R₂ is a group of formula (II) 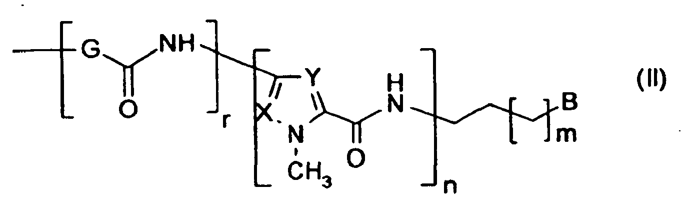 wherein
m is 0;
n is 4;
ris0;
X and Y are both a CH group;
B is the group 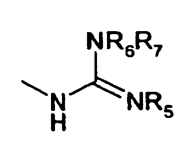 wherein R₅, R₆ and R₇, the same or different, are hydrogen or C₁-C₄ alkyl; or a pharmaceutically acceptable salt thereof, and
- an antineoplastic topoisomerase inhibitor of type I or II.

2. A pharmaceutical composition according to claim 1 wherein the topoisomerase inhibitor is a topoisomerase II inhibitor selected from anthracycline derivatives, including doxorubicin, daunorubicin, epirubicin, nemorubicin and idarubicin; epipodophyllotoxin compounds including etoposide and teniposide; anthraquinone derivatives including mitoxantrone and losoxantrone; acridine derivatives including amsacrine and dactinomycin.

3. A pharmaceutical composition according to claim 2 wherein the topoisomerase U inhibitor is doxorubicin or etoposide.

4. A pharmaceutical composition according to claim 1 comprising an acryloyl distamycin derivative, optionally in the form of a pharmaceutically acceptable salt, selected from the group consisting of
1. N-(5-{[(5-{[(5-{[(2-{[amino(imino)methyl]amino}ethyl)amino]carbonyl}-I-methyl-1H-pyrrol-3-yl)amino]carbonyl}-1-methyl-1H-pyrrol-3-yl)amino]carbonyl}-1-methyl-1H-pyrrol-3-yl)-4-[(2-bromoacryloyl)amino]-1-methyl-1H-pyrrole-2-carboxamide hydrochloride;
2. N-(5-{[(5-{[(5-{[(2-{[amino(imino)methyl]amino}ethyl)amino]carbonyl}-1-methyl-1H-pyrrol-3-yl)amino]carbonyl}-1-methyl-1H-pyrrol-3-yl)amino]carbonyl}-1-methyl-1H-pyrrol-3-yl)-4-[(2-chloroacryloyl)amino]-1-methyl-1H-pyrrole-2-carboxamide hydrochloride;

5. Products comprising an acryloyl distamycin derivative of formula (I): 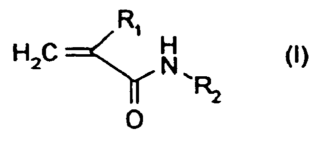 as defined in claim 1; or a pharmaceutically acceptable salt thereof; and an antineoplastic topoisomerase inhibitor of type I or II, as a combined preparation for simultaneous, separate or sequential use in the treatment of tumors.

6. Products according to claim 5 wherein the topoisomerase inhibitor is a topoisomerase II inhibitor selected from anthracycline derivatives, including doxorubicin, daunorubicin, epirubicin, nemorubicin and idarubicin; epipodophyllotoxin compounds including etoposide and teniposide, anthraquinone derivatives including mitoxantrone and losoxantrone; acridine derivatives including amsacrine and dactinomycin.

7. Products according to claim 6 wherein the topoisomerase II inhibitor is doxorubicin or etoposide.

8. Products according to claim 5 wherein the acryloyl distamycin derivative is selected from the group as defined in claim 4.

9. Use of an acryloyl distamycin derivative of formula (I), as defined in claim 1 in the preparation of a medicament for use in combination therapy with an antineoplastic topoisomerase I or II inhibitor in the treatment of tumors.

10. Use according to claim 9 wherein the medicament further comprises the said topoisomerase I or II inhibitor.

11. Use according to claim 9 or 10 wherein the topoisomerase inhibitor is a topoisomerase II inhibitor selected from etoposide or doxorubicin.

12. Use according to claim 9 or 10 wherein the acryloyl distamycin derivative is selected from the group as defined in claim 4.

13. Use according to claim 9 or 10 wherein the tumor is selected from breast, ovary, lung, colon, kidney, stomach, pancreas, liver, melanoma, leukemia and brain tumors.

14. Use of an acryloyl distamycin derivative of formula (I), as defined in claim 1 in the preparation of a medicament for use in combination therapy with an antineoplastic topoisomerase I or II inhibitor in the prevention or treatment of metastasis or in the treatment of tumors by inhibition of angiogenesis.

15. Use according to claim 14 wherein the medicament further comprises the said topoisomerase I or II inhibitor.

16. A pharmaceutical composition according to claim 1 wherein the distamycin derivative optionally in the form of a pharmaceutically acceptable salt, is N-(5-{[(5-{[(5-{[(2-{ [amino(imino)methyl]amino}ethyl)amino]carbonyl}-1-methyl-1H-pyrrol-3-yl)amino]carbonyl }-1-methyl-1H-pyrroi-3-yl)amino]carbonyl }-1-methyl-1H-pyrrol-3-yl)-4-[(2-bromoacryloyl)amino]-1-methyl-1H-pyrrole-2-carboxamide, and wherein the topoisomerase inhibitor is a topoisomerase II inhibitor selected from the group consisting of etoposide or doxorubicin.

## Revendications

1. Composition pharmaceutique comprenant un support ou excipient pharmaceutiquement tolérable et, comme ingrédient actif,
- un dérivé de distamycine acryloyle de la formule (I): 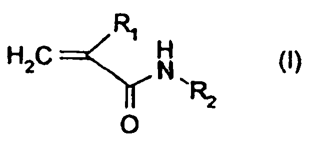 dans laquelle:
R₁ est un atome de brome ou de chlore;
R₂ est un groupe de la formule (II) 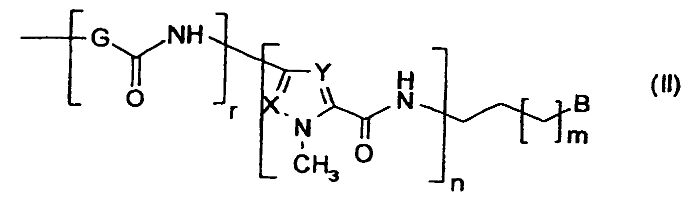
dans laquelle
m est 0;
n est 4;
r est 0;
X et Y sont tous les deux d'un groupe CH;
B est le groupe 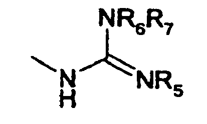 dans lequel R₅, R₆ et R₇, les mêmes ou différents, sont hydrogène ou un alcoyle C₁-C₄; or un sel de celui-ci pharmaceutiquement tolérable; et
- un inhibiteur de la topoisomérase antinéoplastique du type I ou II.

2. Composition pharmaceutique selon la revendication 1 dans laquelle l'inhibiteur de la topoisomérase est un inhibiteur de la topoisomérase II choisi parmi des dérivés d'anthracycline, incluant doxorubicine, daunorubicine, épirubicine, némorubicine et idarubicine; des composés d'épipodophyllotoxine incluant étoposide et téniposide; des dérivés d'anthraquinone incluant mitoxantrone et losoxantrone; des dérivés d'acridine incluant amsacrine et dactinomycine.

3. Composition pharmaceutique selon la revendication 2 dans laquelle l'inhibiteur de la topoisomérase II est doxorubicine ou étoposide.

4. Composition pharmaceutique selon la revendication 1 comprenant un dérivé de distamycine acryloyle, de façon optionnelle sous la forme d'un sel pharmaceutiquement tolérable, choisi parmi le groupe constitué de:
1. N-(5-{[(5-{[(5-{ [(2-{[amino(imino)méthyle]amino}éthyle)amino]carbonyle}-1-méthyle-1H-pyrrole-3-yl)amino]carbonyle}-1-méthyle-1H-pyrrole-3-yl)amino]carbonyle}-1-méthyle-1H-pyrrole-3-yl)-4-[(2-bromoacryloyle)amino]-1-méthyle-1H-pyrrole-2-carboxamide chlorhydrate;
2. N-(5-{[(5-{[(5-{[(2-{[amino(imino)méthyle]amino}éthyle)amino]carbonyle}-1-méthyle-1H-pyrrole-3-yl)amino]carbonyle}-1-méthyle-1H-pyrrole-3-yl)amino]carbonyle}-1-méthyle-1H-pyrrole-3-yl)-4-[(2-chloroacryloyle)amino]-1-méthyle-1H-pyrrole-2-carboxamide chlorhydrate;

5. Produits comprenant un dérivé de distamycine àcryloyle de la formule (I): 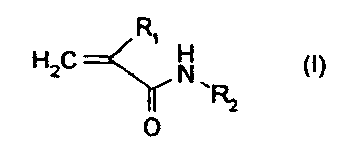 telle que défini dans la revendication 1; ou un sel de celui-ci pharmaceutiquement tolérable; et un inhibiteur de la topoisomérase antinéoplastique du type I ou II, en tant que préparation combinée pour une utilisation simultanée, séparée ou séquentielle dans le traitement des tumeurs.

6. Produits selon la revendication 5 dans lesquels l'inhibiteur de la topoisomérase est un inhibiteur de la topoisomérase II choisi parmi des dérivés d'anthracycline, incluant doxorubicine, daunorubicine, épirubicine, némorubicine et idarubicine; des composés d'épipodophyllotoxine incluant étoposide et téniposide; des dérivés d'anthraquinone incluant mitoxantrone et losoxantrone; des dérivés d'acridine incluant amsacrine et dactinomycine.

7. Produits selon la revendication 6 dans lesquels l'inhibiteur de la topoisomérase II est doxorubicine ou étoposide.

8. Produits selon la revendication 5 dans lesquels le dérivé de distamycine acryloyle est choisi parmi le groupe défini dans la revendication 4.

9. Utilisation d'un dérivé de distamycine acryloyle de la formule (I), telle que défini dans la revendication 1 dans la préparation d'un médicament pour l'utilisation dans une thérapie en combinaison avec un inhibiteur de la topoisomérase antinéoplastique I ou II dans le traitement des tumeurs.

10. Utilisation selon la revendication 9 dans laquelle le médicament comprend en outre ledit inhibiteur de la topoisomérase I ou II.

11. Utilisation selon la revendication 9 ou 10 dans laquelle l'inhibiteur de la topoisomérase est un inhibiteur de la topoisomérase II choisi parmi étoposide ou doxorubicine.

12. Utilisation selon la revendication 9 ou 10 dans laquelle le dérivé de distamycine acryloyle est choisi parmi le groupe défini dans la revendication 4.

13. Utilisation selon la revendication 9 ou 10 dans laquelle la tumeur est choisie parmi les tumeurs du sein, de l'ovaire, du poumon, du colon, du rein, de l'estomac, du pancréas, du foie, au cerveau, le mélanome et la leucémie.

14. Utilisation d'un dérivé de distamycine acryloyle de la formule (I), telle que défini dans la revendication 1 dans la préparation d'un médicament pour l'utilisation dans une thérapie en combinaison avec un inhibiteur de la topoisomérase antinéoplastique I ou II dans la prévention ou le traitement de métastases ou dans le traitement de tumeurs par inhibition d'angiogenèse.

15. Utilisation selon la revendication 14 dans laquelle le médicament comprend en outre ledit inhibiteur de la topoisomérase I ou II.

16. Composition pharmaceutique selon la revendication 1 dans laquelle le dérivé de distamycine, de façon optionnelle sous la forme d'un sel pharmaceutiquement tolérable, est N-(5-{[(5-{[(5-{[(2-{[amino(imino)méthyle]amino}éthyle)amino]carbonyle}-1-méthyle-1H-pyrrole-3-yl)amino]carbonyle}-1-méthyle-1H-pyrrole-3-yl)amino]carbonyle}-1-méthyle-1H-pyrrole-3-yl)-4-[(2-bromoacryloyle)amino]-1-méthyle-H-pyrrole-2-carboxamide, et dans laquelle l'inhibiteur de la topoisomérase est un inhibiteur de la topoisomérase II choisi parmi le groupe contstitué d'étoposide ou doxorubicine.

## Patentansprüche

1. Eine pharmazeutische Zusammensetzung, umfassend einen pharmazeutisch verträglichen Träger oder Bindemittel und, als einen aktiven Bestandteil
- ein Acryloyldistamycinderivat mit der Formel (I): 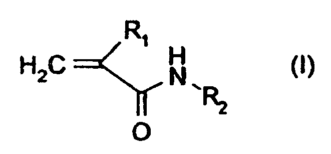 worin:
R₁ ein Brom- oder Chloratom ist;
R₂ ist eine Gruppe der Formel (II) 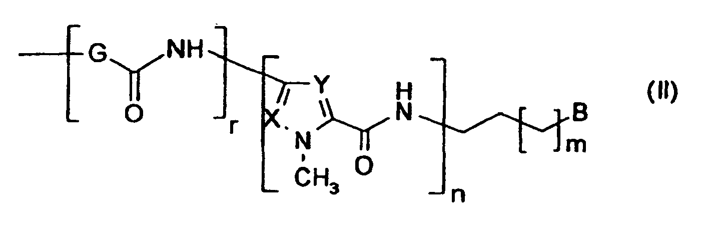 worin
m 0 ist;
n 4 ist;
r 0 ist;
X und Y sind beide eine CH-Gruppe;
B ist die Gruppe 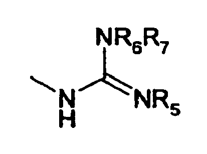 worin R₅, R₆ und R₇, gleich oder unterschiedlich, Wasserstoff oder C₁-C₄ Alkyl sind; oder ein pharmazeutisch verträgliches Salz davon; und
- einen antineoplastischen Topoisomerasehemmer von Typ I oder II.

2. Eine pharmazeutische Zusammensetzung gemäß Anspruch 1, worin der Topoisomerasehemmer ein Topoisomerase-II-hemmer ist, gewählt aus Anthracyclinderivaten, einschließlich Doxorubicin, Daunorubicin, Epirubicin, Nemorubicin und Idarubicin; Epipodophyllotoxinverbindungen, einschließlich Etoposid und Teniposid; Anthrachinonderivaten, einschließlich Mitoxantron und Losoxantron; Acridinderivaten, einschließlich Amsacrin und Dactinomycin.

3. Eine pharmazeutische Zusammensetzung gemäß Anspruch 2, worin der Topoisomerase-II-hemmer Doxorubicin oder Etoposid ist.

4. Eine pharmazeutische Zusammensetzung gemäß Anspruch 1, umfassend ein Acryloyldistamycinderivat, wahlweise in der Form eines pharmazeutisch verträglichen Salzes, gewählt aus der Gruppe bestehend aus:
1. N-(5-{[(5-{[(5-{[(2-{[Amino(imino)methyl]amino}ethyl) amino]carbonyl}-1-methyl-1H-pyrrol-3-yl)amino]carbonyl}-1-methyl-1H-pyrrol-3-yl)amino]carbonyl}-1-methyl-1H-pyrrol-3-yl)-4-[(2-bromacryloyl)amino]-1-methyl-1H-pyrrol-2-carboxamidhydrochlorid;
2. N-(5-{[(5-{[(5-{[(2-{[Amino(imino)methyl]amino}ethyl) amino]carbonyl}-1-methyl-1H-pyrrol-3-yl)amino]carbonyl}-1-methyl-1H-pyrrol-3-yl)amino]carbonyl}-1-methyl-1H-pyrrol-3-yl)-4-[(2-chloracryloyl)amino]-1-methyl-1H-pyrrol-2-carboxamidhydrochlorid;

5. Produkte umfassend ein Acryloyldistamycinderivat mit der Formel (I): 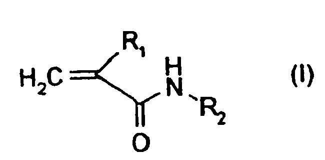 wie in Anspruch 1 definiert; oder ein pharmazeutisch verträgliches Salz davon; und einen antineoplastischen Topoisomerasehemmer von Typ I oder II, als eine kombinierte Zubereitung für die gleichzeitige, getrennte oder aufeinanderfolgende Verwendung in der Behandlung von Tumoren.

6. Produkte gemäß Anspruch 5, worin der Topoisomerasehemmer ein Topoisomerase-II-hemmer ist, gewählt aus Anthracyclinderivaten, einschließlich Doxorubicin, Daunorubicin, Epirubicin, Nemorubicin und Idarubicin; Epipodophyllotoxinverbindungen, einschließlich Etoposid und Teniposid; Anthrachinonderivaten, einschließlich Mitoxantron und Losoxantron; Acridinderivaten, einschließlich Amsacrin und Dactinomycin.

7. Produkte gemäß Anspruch 6, worin der Topoisomerase-II-hemmer Doxorubicin oder Etoposid ist.

8. Produkte gemäß Anspruch 5, worin das Acryloyldistamycinderivat gewählt ist aus der Gruppe wie in Anspruch 4 definiert.

9. Verwendung eines Acryloyldistamycinderivats der Formel (I), wie in Anspruch 1 definiert, in der Herstellung eines Medikaments für die Verwendung in der Kombinationstherapie mit einem antineoplastischen Topoisomerase-I- oder -II-hemmer in der Behandlung von Tumoren.

10. Verwendung gemäß Anspruch 9, worin das Medikament weiter den Topoisomerase-I- oder -II-hemmer umfaßt.

11. Verwendung gemäß Anspruch 9 oder 10, worin der Topoisomerasehemmer ein Topoisomerase-II-hemmer gewählt aus Etoposid oder Doxorubicin ist.

12. Verwendung gemäß Anspruch 9 oder 10, worin das Acryloyldistamycinderivat gewählt ist aus der Gruppe wie in Anspruch 4 definiert.

13. Verwendung gemäß Anspruch 9 oder 10, worin der Tumor gewählt ist aus Brust-, Eierstock-, Lungen-, Dickdarm-, Nieren-, Magen-, Bäuchspeicheldrüsen-, Leber-, Melanom, Leukämie und Gehirntumoren.

14. Verwendung eines Acryloyldistamycinderivats der Formel (I), wie in Anspruch 1 definiert, in der Herstellung eines Medikaments für die Verwendung in der Kombinationstherapie mit einem antineoplastischen Topoisomerase-I- oder -II-hemmer in der Vorbeugung oder Behandlung von Metastasen oder in der Behandlung von Tumoren durch Hemmung der Angiogenese.

15. Verwendung gemäß Anspruch 14, worin das Medikament weiter den Topoisomerase-I- oder -II-hemmer umfaßt.

16. Eine pharmazeutische Zusammensetzung gemäß Anspruch 1, worin das Distamycinderivat, wahlweise in der Form eines pharmazeutisch verträglichen Salzes, N-(5-{[(5-{[(5-{[(2-{[Amino(imino)methyl]amino}ethyl)amino]carbonyl}-1-methyl-1-H-pyrrol-3-yl)amino]carbonyl}-1-methyl-1H-pyrrol-3-yl)amino]carbonyl}-1-methyl-1H-pyrrol-3-yl)-4-[(2-bromacryloyl)amino]-1-methyl-1H-pyrrol-2-carboxamid ist, und worin der Topoisomerasehemmer ein Topoisomerase-II-hemmer gewählt aus der Gruppe bestehend aus Etoposid oder Doxorubicin ist.
